(19) Europäisches Patentamt
European Patent Office
Office européen des brevets

(11) EP 2 035 355 B1

(12) EUROPÄISCHE PATENTSCHRIFT

(45) Veröffentlichungstag und Bekanntmachung des Hinweises auf die Patenterteilung:
**12.03.2014 Patentblatt 2014/11**

(21) Anmeldenummer: **07729915.4**

(22) Anmeldetag: **06.06.2007**

(51) Int Cl.:
***B01J 31/22*** *(2006.01)* ***C07B 37/02*** *(2006.01)*
***C07B 63/04*** *(2006.01)* ***C07C 2/74*** *(2006.01)*

(86) Internationale Anmeldenummer:
**PCT/EP2007/055540**

(87) Internationale Veröffentlichungsnummer:
**WO 2008/003559 (10.01.2008 Gazette 2008/02)**

(54) **VERFAHREN ZUR HERSTELLUNG VON DIENEN DURCH HYDRODIMERISIERUNG**

METHOD FOR PRODUCING DIENES BY HYDRODIMERIZATION

PROCÉDÉ DE PRODUCTION DE DIÈNES PAR HYDRODIMÉRISATION

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **05.07.2006 DE 102006031413**
**18.05.2007 DE 102007023515**

(43) Veröffentlichungstag der Anmeldung:
**18.03.2009 Patentblatt 2009/12**

(73) Patentinhaber: **Evonik Degussa GmbH**
**45128 Essen (DE)**

(72) Erfinder:
- **BREHME, Volker**
**48301 Nottuln-Appelhülsen (DE)**
- **NEUMANN, Manfred**
**45770 Marl (DE)**
- **BAUER, Frank**
**verstorben (DE)**
- **RÖTTGER, Dirk**
**2000 Antwerpen (BE)**

(56) Entgegenhaltungen:
**DE-A1- 10 149 347 DE-A1-102005 036 038**
**US-A- 5 345 007**

- **KIYOTOMI KANEDA ET AL.: "Selective Telomerisation of Butadiene with Various Nucleophiles Catalyzed by Polymer-Bound Palladium(0) Complexes" J. ORG. CHEM., Bd. 46, Nr. 11, 1981, Seiten 2356-2362, XP002450848**
- **HARKAL, SURENDRA ET AL: "Development of a Highly Selective and Efficient Catalyst for 1,3-Butadiene Dimerization" ORGANIC LETTERS, Bd. 7, Nr. 4, 2005, Seiten 541-544, XP002450849**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 035 355 B1

## Beschreibung

Die Erfindung betrifft ein Verfahren zur Herstellung von substituierten oder unsubstituierten 1,7-Diolefinen durch Hydrodimerisierung von nicht-cyclischen Olefinen mit mindestens zwei konjugierten Doppelbindungen in Gegenwart eines Reduktionsmittels und eines Metall-Carben-Komplexes.

1,7-Octadien wird als Co-Monomer oder zur nachträglichen Vernetzung von Polyolefinen für die Produktion von Kunststoffen verwendet. Darüber hinaus stellen substituierte oder unsubstituierte 1,7-Diolefine wertvolle Ausgangsstoffe für die Produktion von $\alpha,\omega$-Diolen bzw. $\alpha,\omega$-Diaminen oder Sebacinsäure dar, die wiederum für die Herstellung von Polyestern bzw. Polyamiden oder Alkydharzen von Interesse sind. Weiterhin dient 1,7-Octadien als Edukt für die Herstellung von 1-Octen - einem Co-Monomer für die Kunststoffherstellung.

Verfahren zur Herstellung von 1,7-Octadien aus 1,3-Butadien mittels Hydrodimerisierung beschreiben zahlreiche Veröffentlichungen.

Gardner et al. beschreiben in Tetrahedron Lett. 2 (1972) 163-164 die Umsetzung von 1,3-Butadien mit Ameisensäure in Gegenwart verschiedener Katalysatoren, wie beispielsweise Diacetopalladium(II) ($Pd(OAc)_2$), $Li_2PtCl_4$ oder Palladiumkomplexe mit Phosphin-Liganden, zu 1,6-Octadien. Ebenfalls wird beschrieben, dass bei einer Umsetzung von 1,3-Butadien mit Ameisensäure in Dimethylformamid und in Gegenwart eines $Li_2PtCl_4$-Katalysators bis zu 80 % an 1,7-Octadien entstehen.

Roffia et al. beschreiben in J. Organomet. Chem. 55 (1973) 405-407 ebenfalls die Reaktion von 1,3-Butadien mit Ameisensäure. Als Katalysatorprecursor wird $(PPh_3)PdCl_2$ eingesetzt, welcher unter den genannten Reaktionsbedingungen reduziert wird. Als Produkt wird eine Mischung aus 1,3,7-Octatrien, 1,7-Octadien und 1,6-Octadien erhalten.

In J. Mol. Catal., 15 (1982) 377 - 381 beschreiben Pittmann et al. die Verwendung von Katalysatoren auf Basis von Palladium mit Phosphin-Liganden zur Herstellung von 1,7-Octadien. Die höchste Selektivität von 93 % bezogen auf 1,7-Octadiene wird hierbei bei der Verwendung von Triethylphosphin-Liganden erzielt.

EP 0 004 408 B1 und EP 0 004 410 B1 beschreiben ein Verfahren zur Herstellung von 1,7-Octadien aus 1,3-Butadien in Gegenwart eines Palladium-Phosphin-Katalysators. Die EP 0 004 410 beschreibt zusätzlich den Einsatz von Palladium, Platin oder Rhodium auf einem inerten Trägermaterial als Co-Katalysator.

EP 0 007 666 B1 beschreibt ebenfalls ein Verfahren zur Herstellung von 1,7-Octadien aus 1,3-Butadien in Gegenwart eines Palladium-Phosphin-Katalysators, dieses Verfahren zeichnet sich durch den Einsatz von Formiaten als Reduktionsmittel aus.

EP 0 012 472 und EP 0 012 475 offenbaren ein Verfahren, in dem 1,7-Octadien in Gegenwart eines Palladiumkatalysators und tertiären Organophosphorverbindungen, wie beispielsweise Organophosphoniten bzw. Organophosphiniten oder Mischungen hieraus, aus 1,3-Butadien und Ameisensäure bzw. Formiaten hergestellt wird.

EP 0 008 139 B1 beschreibt ein Verfahren zur Herstellung von 1,7-Octadien aus 1,3-Butadien in Gegenwart eines Palladiumkatalysators, tertiären Phosphinen und eines Lösungsmittels, ausgewählt aus Dialkylsulfoxide bzw. Dialkylformamide, in Abwesenheit einer Base.

Die beiden Offenlegungsschriften DE 30 24 877 A1 und DE 30 24 878 A1 beschreiben ein Verfahren zu Hydrodimerisierung von 1,3-Butadien zum 1,7-Octadien mittels eines Palladium-Phosphin-Katalysators, wobei als Reduktionsmittel Wasserstoff oder eine Wasserstoff Kohlendioxid-Mischung eingesetzt wird.

Die Offenlegungsschrift DE 30 24 879 A1 beschreibt ein Verfahren zu Hydrodimerisierung von 1,3-Butadien zum 1,7-Octadien mittels eines Palladium-Phosphin-Katalysators und einem Reduktionsmittel, wobei als Lösungsmittel ein sauerstoffhaltiger Stickstoffheterocyclus eingesetzt wird. Nach der destillativen Abtrennung des 1,7-Octadiens wird der katalysatorhaltige Destillationssumpf der Hydrodimerisierung zurückgeführt.

Die Offenlegungsschrift DE 30 34 098 A1 beschreibt ein Verfahren zur Herstellung von Alkadienen durch Hydrodimerisierung von 1,3-Butadien oder Isopren in Gegenwart eines Palladium- oder Platin-Katalysators in einer Sulfolanlösung, wobei die Reaktionsmischung tertiäre Alkylaminformiate und Phosphinverbindungen aufweist. Auch hier wird der Katalysator nach der Hydrodimerisierung abgetrennt und anschließend der Hydrodimerisierung zurückgeführt.

JP 09-087207 A beschreibt die Dimerisierung von Alkadienen, wie beispielsweise 1,3-Butadien, in Gegenwart eines Reduktionsmittels und eines Palladium-Phosphinit-Katalysators. Hierbei werden Ausbeuten von 94,7 % an Octadien erzielt, wobei die Reinheit des 1,7-Octadiens 97,1 % beträgt.

EP 0 704 417 A2 beschreibt ein Verfahren zur Herstellung von Octadienen aus 1,3-Butadien und Ameisensäure in Gegenwart eines Palladiumkatalysators mit phosphorhaltigen Liganden, wobei der Druck des Systems nicht über dem Dampfdruck des Butadiens liegt. Das Mengenverhältnis von 1,7-Octadien zu 1,6-Octadien im Produkt beträgt 89 zu 11.

DE 101 49 347 A1 offenbart ein Verfahren zur Herstellung von 1-Octen in einem zweistufigen Prozess. Im ersten Schritt wird 1,3-Butadien in Gegenwart eines Telomerisationskatalysators und eines Reduktionsmittels zu 1,7-Octadien umgesetzt, im zweiten Schritt wird das 1,7-Octadien zum 1-Octen partiell hydriert. Als Telomerisationskatalysator werden zahlreiche Nickel-, Rhodium-, Palladium- und Platinverbindungen beschrieben, u.a. werden auch Pd(0)-Verbindungen mit Carbenliganden genannt. Als geeignete Carbenliganden werden 1,3-disubstituierte 2,3-Dihydro-1H-imidazol-2-ylidene, 1,3-disubstituierte 4,5-Dihydro-1H-triazol-5-ylidene und gegebenenfalls 2,3-Dihydrothiazol-2-ylidene genannt.

WO 2005/047217 und WO 2005/047218 offenbaren jeweils ein Verfahren zur Herstellung von 1-Octen in einem zweistufigen Prozess. Im ersten Verfahrensschritt wird 1,3-Butadien in Gegenwart eines Palladiumkomplexes, der einen oder mehrere trisubstituierte, monodentate Phosphorverbindungen enthält, und eines Wasserstoffdonors in einem aprotischen, polaren Lösemittel zu 1,7-Octadien umgesetzt.

DE 101 28 144 A1 offenbart ein Verfahren zur Telomerisation von nicht-cyclischen Olefinen mit mindestens zwei konjugierten Doppelbindungen mit Nukleophilen, wobei als Katalysator ein Palladiumcarbenkomplex eingesetzt wird.

Unter einer Telomerisation wird die Umsetzung von Olefinen mit konjugierten Doppelbindungen in Gegenwart von Nukleophilen verstanden. Als Produkte entstehen Verbindungen, die sich aus zwei Äquivalenten des Diens, sowie einem Äquivalent des Nukleophils aufbauen, wobei das Produkt ein Gemisch aus mehreren Verbindungen darstellt - wie das Reaktionsschema 1 zeigt.

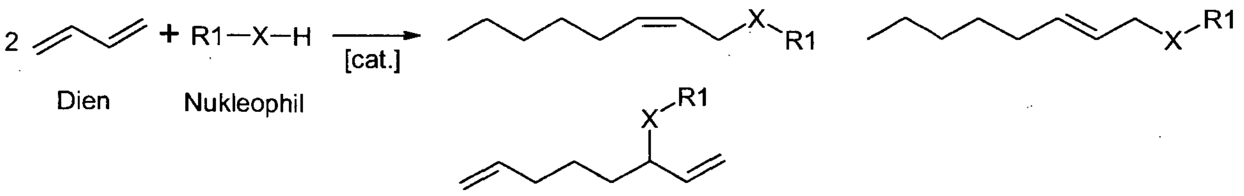


**Reaktionsschema 1**

Als Hauptprodukte einer Telomerisation entstehen 1-substituierte 2,7-Octadien, als Nebenprodukte entstehen hierbei 3-substituierte 1,7-Octadiene. Bei Verwendung von Telomerisationskatalysatoren, die mit Carbenliganden modifiziert sind, werden Verhältnisse von Haupt- zu Nebenprodukt von bis zu 98 zu 2 erreicht (Chem. Eur. J. 10 (2004) 3891-3900).

Im Gegensatz hierzu erfolgt bei einer Hydrodimerisierung die Umsetzung von zwei Äquivalenten eines Olefins mit konjugierten Doppelbindungen in Gegenwart eines Reduktionsmittels gemäß dem Reaktionsschema 2:

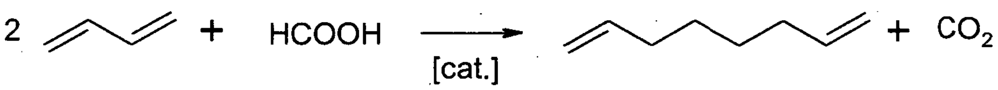


**Reaktionsschema 2**

Das bei einer Hydrodimerisierung eingesetzte Reduktionsmittel dient als Wasserstofflieferant, im Falle der Ameisensäure als Reduktionsmittel entsteht als Koppelprodukt Kohlendioxid. Hauptprodukt der Hydrodimerisierung von 1,3-Butadien ist 1,7-Octadien.

Die Verfahren der Hydrodimerisierung von 1,3-Butadien zum 1,7-Octadien in Gegenwart von Phosphor-modifizierten Palladiumkatalysatoren weisen geringe Produktivität (TON), Aktivität (TOF) und/oder Selektivität auf, wobei diese wie folgt definiert sind:

$$\text{TON} = \text{n(Produkt)} / \text{n(Katalysator)} \quad \text{bzw.}$$

$$\text{TOF} = \text{n(Produkt)} / [\text{n(Katalysator)} * \text{Zeit}]$$

$$\text{Selektivität} = \text{n(Produkt)} / [\text{n(Produkt)} + \text{n(Nebenprodukte)}].$$

Generell sind dreiwertige Phosphorverbindungen sauerstoffempfindlich und werden leicht zur entsprechenden fünfwertigen Phosphorspezies oxidiert, wodurch eine Deaktivierung des Katalysators einhergeht. Die Rückführung dieser Phosphor-modifizierten Palladiumkatalysatoren ist aufgrund ihrer hohen Empfindlichkeit schwierig und technisch nur mit großen Aktivitätsverlusten durchführbar. Palladium(II)-Phosphin-Komplexe werden unter den Reaktionsbedingungen der Hydrodimerisierung reduziert und es entstehen thermisch instabile Zwischenstufen. Werden Palladiumkomplexe mit Trialkylphosphinen modifiziert, so entstehen aktive und bezüglich des 1,7-Octadiens selektive Katalysatoren. Trialkylphosphine sind allerdings pyrophor, teuer und im technischen Maßstab schwierig einsetzbar. Die besser handzuhabenden Triarylphosphine zeigen im Vergleich zu den Trialkylphosphinen bei der Hydrodimierisierung eine deutlich schlechtere Selektivität. In den Verfahren gemäß dem Stand der Technik werden diese Phosphinverbindungen der Hydrodimerisierung im Überschuss zugegeben. Auch bei der Handhabung von Phosphoniten und Phosphiniten in großtechnischen Anlagen sind Vorsichtsmaßnahmen zu treffen, da diese Verbindungen hydrolyse- und säureempfindlich sind. Die Verwendung von Ameisensäure bzw. Ameisensäurederivaten bei der Hydrodimerisierung wirkt sich dementsprechend negativ auf die Stabilität von phosphonit- bzw. phosphinit-modifizierte Katalysatoren aus.

Es war deshalb die Aufgabe der vorliegenden Erfindung ein Verfahren zur Herstellung von substituierten oder unsubstituierten 1,7-Diolefinen durch Hydrodimerisierung zur Verfügung zu stellen, welches sich durch eine vereinfachte Handhabung des Katalysators als auch durch eine hohe Katalysatorproduktivität auszeichnet. Insbesondere sollten hohe Selektivitäten bezüglich des substituierten oder unsubstituierten 1,7-Octadiens erreicht werden.

Überraschenderweise wurde gefunden, dass substituierte oder unsubstituierte 1,7-Diolefine durch Hydrodimerisierung von nicht-cyclischen Olefinen mit mindestens zwei konjugierten Doppelbindungen in Gegenwart eines Reduktionsmittels und eines Katalysators nach einem neuen Verfahren hergestellt werden können, welches sich dadurch auszeichnet, dass als Katalysator ein Metall-Carben-Komplex eingesetzt wird, der ein Metall der 8. bis 10. Gruppe des Periodensystems und mindestens einen Carben-Liganden aufweist. Das erfindungsgemäße Verfahren weist gegenüber Verfahren gemäß dem Stand der Technik eine verbesserte Selektivität als auch eine höhere Produktivität auf. Bei dem erfindungsgemäßen Verfahren können beispielsweise bei der Hydrodimerisierung von 1,3-Butadien mit Ameisensäuren Katalysatorproduktivitäten von größer 10000 realisiert werden. Insbesondere zeigte sich, dass bei dem Einsatz von Stabilisatoren für die nicht-cyclischen Olefine, ausgewählt aus alkylierten Phenolen und stabilen N-Oxylradikalen, geringere Mengen an Katalysator im Vergleich zur Verwendung von 4-tert-Butylkatechol- dem Stabilisator gemäß dem Stand der Technik - benötigt werden. Weiterhin zeichnet sich diese Verfahrensvariante des erfindungsgemäßen Verfahrens durch eine geringe Nebenproduktbildung aus. Das erfindungsgemäße Verfahren weist somit eine höhere Verfahrenssicherheit gegenüber Verfahren gemäß dem Stand der Technik als auch eine hohe Katalysatorproduktivität auf.

Gegenstand der Erfindung ist ein Verfahren zur Herstellung von substituierten oder unsubstituierten 1,7-Diolefinen durch Hydrodimerisierung von nicht-cyclischen Olefinen mit mindestens zwei konjugierten Doppelbindungen in Gegenwart eines Reduktionsmittels und eines Katalysators, das dadurch gekennzeichnet ist, dass als Katalysator ein Metall-Carben-Komplex eingesetzt wird, der ein Metall der 8. bis 10. Gruppe des Periodensystems und mindestens einen Carben-Liganden gemäß der Struktur **1, 2, 3** oder **4**

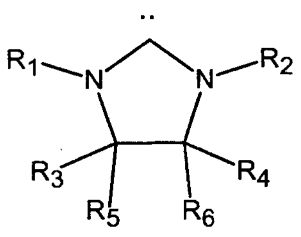
**(1)**

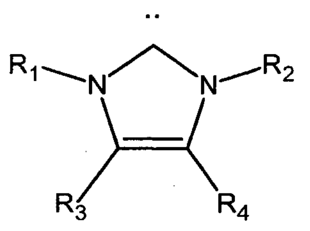
**(2)**

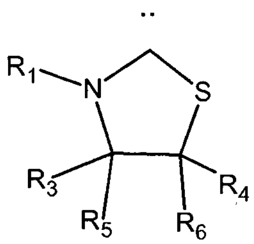
**(3)**

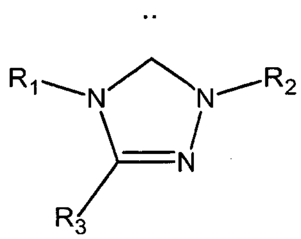
**(4)**

mit:

$R_1$, $R_2$ = $-(CH_2)_n-B$
B = mono- oder polycyclische Arylgruppe mit 6 bis 14 Kohlenstoffatomen oder mono- oder polycyclischer Heterocyclus mit 5 bis 14 Kohlenstoff und Heteroatomen, wobei dieser Heterocyclus 1 bis 3 Heteroatome, ausgewählt aus der Gruppe N, O und S, aufweist,

n =0-4

$R_3$, $R_4$, $R_5$ und $R_6$ = Wasserstoff, Alkyl-, Heteroaryl-, Aryl-, -CN, -COOH, -COO-Alkyl-, -COO-Aryl-, -OCO-Alkyl-, OCO-Aryl-, -OCOO-Alkyl-, OCOO-Aryl-, -CHO, -CO-Alkyl-, -CO-Aryl-, $-NH_2$, -NH(Alkyl)-, $-N(Alkyl)_2$-, NH(Aryl)-, -N $(Aryl)_2$-, -F,-Cl, -Br, -I, -OH, $-CF_3$, $-NO_2$, -Ferrocenyl, $-SO_3H$, $-PO_3H_2$, wobei die Alkylgruppen von 1 bis 12 und die Arylgruppen von 5 bis 14 Kohlenstoffatome aufweisen und die Substituenten vom Typ $R_3$ und $R_4$ auch Teil eines verbrückenden aliphatischen oder aromatischen Ringes sein können,

aufweist und die Substituenten vom Typ $R_1$ und $R_2$ gleich oder verschieden sowie substituiert oder unsubstituiert sind, und die Substituenten vom Typ $R_3$, $R_4$, $R_5$ und $R_6$ ebenfalls gleich oder verschieden sowie substituiert oder unsubstituiert sind und als Reduktionsmittel Ameisensäure und/oder Formiate eingesetzt werden.

Das erfindungsgemäße Verfahren zur Herstellung von substituierten oder unsubstituierten 1,7-Diolefinen durch Hydrodimerisierung von nicht-cyclischen Olefinen mit mindestens zwei konjugierten Doppelbindungen in Gegenwart eines Reduktionsmittels und eines Katalysators, zeichnet sich dadurch aus, dass als Katalysator ein Metall-Carben-Komplex eingesetzt wird, der ein Metall der 8. bis 10. Gruppe des Periodensystems und mindestens einen Carben-Liganden gemäß der Struktur **1, 2, 3** oder **4**

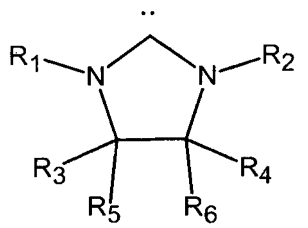

(1)

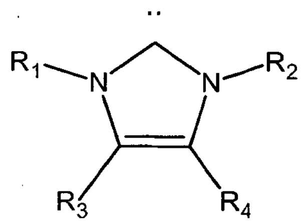

(2)

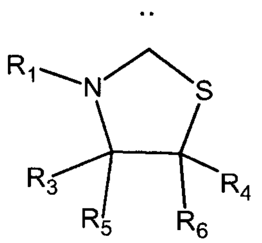

(3)

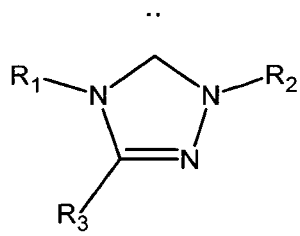

(4)

mit:

| | |
|---|---|
| $R_1$, $R_2$ | = $-(CH_2)_n$-B |
| B | = mono- oder polycyclische Arylgruppe mit 6 bis 14 Kohlenstoffatomen oder mono- oder polycyclischer Heterocyclus mit 5 bis 14 Kohlenstoff- und Heteroatomen, wobei diese Heterocyclus 1 bis 3 Heteroatome, ausgewählt aus der Gruppe N, O und S, aufweist, bevorzugt jedoch monocyclische Arylgruppe mit 6 Kohlenstoffatomen, |
| n | = 0 - 4, bevorzugt von 0 - 1, besonders bevorzugt 0 |
| $R_3$, $R_4$, $R_5$ und $R_6$ | = Wasserstoff, Alkyl-, Heteroaryl-, Aryl-, -CN, -COOH, -COO-Alkyl-, -COO-Aryl-, -OCO-Alkyl-, OCO-Aryl-, -OCOO-Alkyl-, OCOO-Aryl-, -CHO, =CO-Alkyl-, -CO-Aryl-, $-NH_2$, -NH(Alkyl)-, $-N(Alkyl)_2$-, NH(Aryl)-, $-N(Aryl)_2$-, -F, -Cl, -Br, -I, -OH, $-CF_3$, $-NO_2$, -Ferrocenyl,$-SO_3H$, $-PO_3H_2$, wobei die Alkylgruppen von 1 bis 12 und die Arylgruppen von 5 bis 14 Kohlenstoffatome aufweisen und die Substituenten vom Typ $R_3$ und $R_4$ auch Teil eines verbrückenden aliphatischen oder aromatischen Ringes sein können, |

aufweist und die Substituenten vom Typ $R_1$ und $R_2$ gleich oder verschieden sowie substituiert oder unsubstituiert sind, und die Substituenten vom Typ $R_3$, $R_4$, $R_5$ und $R_6$ ebenfalls gleich oder verschieden sowie substituiert oder unsubstituiert sind.

Im Rahmen dieser Erfindung werden unter substituierten oder unsubstituierten 1,7-Diolefine Verbindungen gemäß der Struktur 9 verstanden:

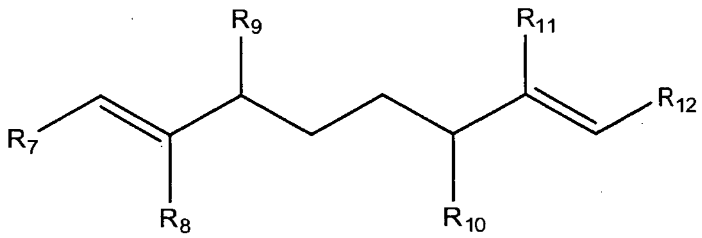

(9)

wobei $R_7$ bis $R_{11}$ unabhängig von einander eine Alkylgruppe, insbesondere weisen diese von 1 bis 4 Kohlenstoffatome, bevorzugt ein Kohlenstoffatom, auf, oder eine Halogengruppe, insbesondere Chlor, ist.

In einer besonderen Ausführungsform des erfindungsgemäßen Verfahrens weisen die Metall-Carben-Komplexe Carben-Liganden mit Substituenten vom Typ $R_1$, $R_2$, $R_3$, $R_4$, $R_5$ oder $R_6$ auf, die mindestens einen Substituenten aus der Gruppe H, -CN, -COOH, -COO-Alkyl-, - COO-Aryl-, -OCO-Alkyl-, OCO-Aryl-, -OCOO-Alkyl-, OCOO-Aryl-, -CHO, -CO-Alkyl-, - CO-Aryl-, $-NH_2$, -NH(Alkyl)-, $-N(Alkyl)_2-$, NH(Aryl)-, $-N(Aryl)_2-$, -F, -Cl, -Br, -I, -OH,$-CF_3$, $-NO_2$, -Ferrocenyl, $-SO_3H$, $-PO_3H_2$ aufweisen, wobei die Alkylgruppen 1 bis 12 und die Arylgruppen 5 bis 14 Kohlenstoffatome beinhalten.

Bevorzugt werden in dem erfindungsgemäßen Verfahren Metall-Carben-Komplexe eingesetzt, die ein Metall, ausgewählt aus Nickel, Rhodium, Palladium und/oder Platin, aufweisen. Besonders bevorzugt werden jedoch Metall-Carben-Komplexe eingesetzt, die Palladium als Metall aufweisen.

Im Rahmen dieser Erfindung werden unter Carbenliganden sowohl freie Carbene, die als Liganden fungieren können, als auch an das Metall koordinierte Carbene verstanden. In dem erfindungsgemäßen Verfahren werden vorzugsweise Metall-Carben-Komplexe eingesetzt, die zumindest ein Carben-Liganden, ausgewählt aus den Strukturen **1** bis **4** mit $R_3$ bis $R_6$ = Wasserstoff, aufweisen. Bevorzugt weisen die eingesetzten Metall-Carben-Komplexe zumindest einen Carben-Liganden gemäß der Struktur **2** mit $R_3$ und $R_4$ gleich Wasserstoff auf. Besonders bevorzugt werden Metall-Carben-Komplexe eingesetzt, die zumindest ein Carben-Liganden gemäß der Struktur **2** mit $R_3$ und $R_4$ gleich Wasserstoff und $R_1$ und $R_2$ mit n = 0 und B gleich einer Phenyl- oder 2,4,6-Trimethylphenylgruppe aufweisen.

Beispiele für Carbenliganden, die den allgemeinen Strukturen **1** oder **2** entsprechen, und Komplexe, die derartige Liganden enthalten, sind in der Fachliteratur bereits beschrieben (W. A. Herrmann, C. Köcher, Angew. Chem. 1997, 109, 2257; Angew. Chem. Int. Ed. Engl. 1997, 36, 2162; V.P.W. Böhm, C.W.K. Gstöttmayr, T. Weskamp, W.A. Herrmann, J. Organomet. Schem. 2000, 595, 186; DE 44 47 066).

Das Katalysatormetall, wobei es sich hierbei bevorzugt um Palladium handelt und mit dem sich unter den Reaktionsbedingungen die aktiven Katalysatoren bilden, kann auf verschiedene Weisen in das erfindungsgemäße Verfahren eingebracht werden:

a.) als Palladium-Carben-Komplex, wobei das Palladium bevorzugt in den Oxidationsstufen (II) oder (0) vorliegt.

b.) in Form von Precursoren, aus denen in-situ die Katalysatoren gebildet werden. Dies kann durch Erweiterung der Ligandensphäre oder durch Aufbrechen von Brückenstrukturen, ausgehend von Metallsalzen oder Metallkomplexen, geschehen. Eine weitere Möglichkeit ist der Austausch von am Zentralmetall koordinierenden Liganden durch die Carbenliganden. Bevorzugt werden hierbei Palladium(0)- und/oder Palladium(II)-Verbindungen eingesetzt, besonders bevorzugt werden Palladium(II)acetat, Palladium(II)acetylacetonat und/oder Bis(dibenzylidenaceton)palladium(0) eingesetzt. Die Carben-Liganden nach den Strukturen **1** bis **4** werden in Form freier Carbene oder als Metallkomplexe eingesetzt oder in situ aus Carbenvorstufen erzeugt. Als Carbenvorstufen eignen sich beispielsweise Salze der Carbene gemäß den Strukturen **5** bis **8**,

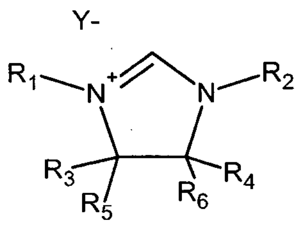

**(5)**

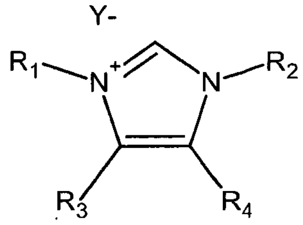

**(6)**

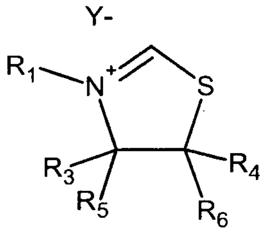 **(7)** 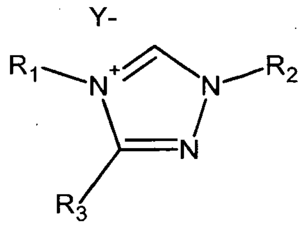 **(8)**

wobei die Substituenten vom Typ $R_1$ bis $R_6$ dieselbe Bedeutung haben wie in den Strukturen **1** bis **4** und Y für eine einfach geladene anionische Gruppe oder entsprechend der Stöchiometrie anteilig für eine mehrfach geladene anionische Gruppe steht.

Beispiele für Y sind Halogenide, Hydrogensulfat, Sulfat, Alkylsulfate, Arylsulfate, Borate, Hydrogencarbonat, Carbonat, Alkylcarboxylate, Arylcarboxylate. Aus den Salzen der Carbene können die entsprechenden Carbene beispielsweise durch Umsetzung mit einer Base freigesetzt werden. Als Basen eignen sich beispielsweise Metallhydride, Metallalkoholate, Carbonylmetallate, Metallcarboxylate, Metallamide oder Metallhydroxide.

Die Menge ist stark abhängig von der Art an eingesetzter Base. Bei Metall-Carben-Komplexen werden vorzugsweise von 0 bis 50 000 Mol Base pro Mol Metall eingesetzt, bevorzugt von 0,5 bis 5000, besonders bevorzugt von 0,5 bis 500 Mol Base pro Mol Metall. Es ist auch möglich, eine Mischung von mehreren Basen einzusetzen.

Die Konzentration des Katalysators, formal angegeben in ppm (Masse) an Metall bezogen auf die Gesamtmasse der Reaktionsmischung, beträgt zu Beginn der Hydrodimerisierung in dem erfindungsgemäßen Verfahren vorzugsweise von 0,01 ppm bis 5000 ppm, bevorzugt von 0,1 bis 1000 ppm, besonders bevorzugt von 0,1 bis 500 ppm und ganz besonders bevorzugt von 1 bis 100 ppm. In einer besonderen Ausführungsform des erfindungsgemäßen Verfahrens kann diese Konzentration des Katalysators von 1 bis 50 ppm betragen. Das Verhältnis [Mol/Mol] von Carben-Ligand zu Metall beträgt vorzugsweise von 0,01:1 bis 250:1, bevorzugt von 0,1:1 bis 100:1, besonders bevorzugt von 1:1 bis 20:1.

Der Carben-Ligand kann der Reaktion in Substanz, gelöst oder in Form von Metallkomplexen zugeführt werden. Ein zusätzlicher Ligand kann der Reaktion zu jedem Zeitpunkt und an beliebiger Stelle im Reaktor in Substanz, als Lösung oder in Form eines Metallkomplexes zugeführt werden. Dieser zusätzliche Ligand kann ebenfalls ein Carben bzw. eine Carbenvorstufe sein, oder aber einer anderen Klasse von Liganden angehören, wie beispielsweise Phosphorliganden, insbesondere Triphenylphosphin. Bevorzugt werden in dem erfindungsgemäßen Verfahren Katalysatoren eingesetzt, die keine phosphorhaltigen Liganden aufweisen, besonders bevorzugt werden Katalysatoren eingesetzt, die ausschließlich Carbenliganden aufweisen und ganz besonders bevorzugt Katalysatoren, die ausschließlich Carbenliganden gemäß den Strukturen **1, 2, 3** oder **4** aufweisen.

Der Katalysator kann nach dem Hydrodimerisierungprozess abgetrennt und für eine erneute Reaktion zurückgeführt werden. Diese Abtrennung kann beispielsweise über eine Destillation, Extraktion, Fällung oder Adsorption erfolgen. Liegt der Katalysator ganz oder teilweise in einer zweiten Phase vor, kann dies durch eine einfache Trennung der Phasen erfolgen.

Aufgrund der Katalysatoraktivitäten und -stabilitäten ist es bei dem erfindungsgemäßen Verfahren möglich, extrem kleine Mengen an Katalysator zu verwenden. Hierdurch besteht die Option, den Katalysator auf Grund der niedrigen Konzentrationen im Reaktionsgemisch, nicht zurückführen zu müssen.

Wird ein Metall-Carben-Komplex eingesetzt, dessen Oxidationszahl > 0 ist, so kann dieser Komplex optional vor der Hydrodimerisierung vorreduziert werden. Das hierfür verwendete Reduktionsmittel kann mit dem bei der Hydrodimerisierung verwendeten Reduktionsmittel identisch sein. Mögliche Reduktionsmittel sind beispielsweise Ameisensäure, Formiate, Wasserstoff, Alkalimetallborhydride, Alkalimetallaluminiumhydride oder Borane.

In dem erfindungsgemäßen Verfahren werden vorzugsweise nicht-cyclische Olefine mit mindestens zwei konjugierten Doppelbindungen, ausgewählt aus 1,3-Butadien, Isopren, 1,3-Pentadien, Chloropren, oder entsprechende Mischungen, die nicht-cyclische Olefine mit mindestens zwei konjugierten Doppelbindungen aufweisen, wie beispielsweise aus einem Crackverfahren entstehen, eingesetzt. Bevorzugt wird in dem erfindungsgemäßen Verfahren 1,3-Butadien eingesetzt.

Die nicht-cyclischen Olefine mit mindestens zwei konjugierten Doppelbindungen können stabilisiert oder nicht stabilisiert eingesetzt werden. Als Stabilisatoren sind vorzugsweise radikalisch wirkende Inhibitoren, wie beispielsweise alkylierte Phenole, alkylierte Katechine oder stabile N-Oxylradikale, geeignet. Insbesondere werden in dem erfindungsgemäßen Verfahren nicht-cyclische Olefine eingesetzt, die entweder keinen Stabilisator oder einen Stabilisator, ausgewählt aus alkylierten Phenolen oder stabilen N-Oxylradikalen, aufweisen. Bevorzugt werden in dem erfindungsgemäßen Verfahren nicht-cyclische Olefine eingesetzt, die als Stabilisator alkylierte Phenole oder stabile N-Oxylradikale aufweisen. Besonders bevorzugt sind stabile N-Oxylradikale als Stabilisator, wie beispielsweise an 4-Position substituierte oder unsubstituierte 2,2,6,6-Tetramethylpiperidin-N-oxyle, insbesondere 4-Hydroxy-2,2,6,6,-tetramethylpiperidin-N-oxyl oder 2,2,6,6-Tetramethylpiperidin-N-oxyl. Der Stabilisator kann in dem erfindungsgemäßen Verfahren auch separat von den

nicht-cyclischen Olefinen der Reaktionsmischung zugegeben werden.

Als Reduktionsmittel können in dem erfindungsgemäßen Verfahren Ameisensäure und/oder Formiate eingesetzt werden, bevorzugt wird Ameisensäure eingesetzt. Beispiele für Formiate sind Ammoniumformiat, organische Salze wie beispielsweise Triethylammoniumformiat, Trimethylammoniumformiat, Tripropylammoniumformiat und Alkali- und Erdalkalimetallsalze, insbesondere Lithiumformiat, Natriumformiat, Kaliumformiat, Magnesiumformiat und Calciumformiat. Die Formiate können in Substanz oder in gelöster Form der Reaktion zugesetzt werden oder in situ dargestellt werden. So sind die Alkalimetallformiate aus der Reaktion von Ameisensäure mit den Metallhydroxiden darstellbar, können aber auch aus Metallhydroxiden und Kohlenmonoxid dargestellt werden. Als Kohlenmonoxidquelle können Kohlenmonoxid enthaltene Gase, beispielsweise Synthesegas ($H_2$/CO-Gemisch), eingesetzt werden.

Zur Bildung eines Mols 1,7-Octadien aus zwei Molen Butadien wird ein Mol Ameisensäure bzw. Formiat benötigt (Stöchiometrie der Reaktion). Je nach Reaktionsführung kann die gesamte Menge und gegebenenfalls ein Überschuss an Reduktionsmittel vollständig zu Reaktionsbeginn zugegeben werden. Alternativ kann das Reduktionsmittel im Verlauf der Reaktion zudosiert werden. Wird in dem erfindungsgemäßen Verfahren Ameisensäure als Reduktionsmittel eingesetzt, so sollte die Ameisensäure im Verlauf der Reaktion zudosiert werden. Wird die Ameisensäure vorgelegt, so kann eine Deaktivierung des Katalysators beobachtet werden.

Es ist möglich, mehrere Reduktionsmittel nebeneinander einzusetzen.

In dem erfindungsgemäßen Verfahren beträgt das Verhältnis [Mol/Mol] zwischen eingesetztem nicht-cyclischen Olefin mit mindestens zwei konjugierten Doppelbindungen und Reduktionsmittel vorzugsweise von 1:1 bis 10:1, bevorzugt von 2:1 bis 4:1 und besonders bevorzugt von 2:1 bis 3:1.

Bei dem erfindungsgemäßen Verfahren werden vorzugsweise Lösungsmittel eingesetzt, die unter den Reaktionsbedingungen inert sind, oder ein weitgehend inertes Verhalten zeigen. Geeignete Lösungsmittel sind unter anderem aliphatische, cycloaliphatische und aromatische Kohlenwasserstoffe, Amine, wie beispielsweise Pyridin und 2,6-Lutidin, Amide wie beispielsweise N-Methylpyrrolidin, Acetamid, N,N-Dimethylformamid, N,N-Dimethylacetamid, Nitrile, wie beispielsweise Acetonitril und Benzonitril, Ketone, wie beispielsweise Aceton und Methylethylketon, Carbonsäureester, wie beispielsweise Essigsäureethylester, Ether, wie beispielsweise Methyl-tert.-butylether, Tetrahydrofuran, Dioxan, Anisol, Alkyl- und Arylether von Ethylenglycol, Diethylenglycol und Polyethylenglycol und andere polare Lösungsmittel, wie beispielsweise Dimethylsulfoxid, 1,3-Dimethyl-2-imidazolidinon, 1,3-Dimethyl-3,4,5,6-tetrahydro-2(1H)-pyrimidinon, Sulfolan, Propylencarbonat oder Tetrahydrothiophenoxid. Auch ionische Flüssigkeiten, beispielsweise Imidazolium- oder Pyridiniumsalze, können als Lösungsmittel eingesetzt werden. Die Lösemittel können allein oder auch als Mischungen verschiedener Lösemittel eingesetzt werden. Bevorzugt werden in dem erfindungsgemäßen Verfahren Lösemittel eingesetzt, die eine relative Dielektrizitätskonstante von 30 bis 100 (bei einer Temperatur von 20 °C) aufweisen, insbesondere Lösemittel, ausgewählt aus N-Methylpyrrolidon, N,N-Dimethylformamid, Dimethylsulfoxid und Dimethylacetamid.

Oftmals ist es vorteilhaft, die Hydrodimerisierung in Gegenwart von Basen durchzuführen. Beispiele für geeignete Basen sind Metallhydroxide, insbesondere Alkalimetallhydroxide und Erdalkalimetallhydroxide, Metallcarbonate und Metallhydrogencarbonate, insbesondere Alkalimetall- und Erdalkalimetallcarbonate und Alkali- und Erdalkalimetallhydrogencarbonate, Hydroxide quartärer Ammonium- oder Phosphoniumionen, Alkoholate, Enolate, Phenolate, Metallsalze von Carbonsäuren, Metallamide, wie zum Beispiel Natriumamid oder Lithiumdiethylamid, und organische Stickstoffbasen, insbesondere Amine, wie zum Beispiel Triethylamin, Trioctylamin oder Pyridin. Auch Kohlendioxid kann als Base eingesetzt werden. Bevorzugt werden Alkalimetallhydoxide, Alkalimetallalkoholate oder tertiäre Amine in dem erfindungsgemäßen Verfahren als Basen eingesetzt.

Die Menge an eingesetzter Base, die der Hydrodimerisierung zugesetzt werden kann, ist stark abhängig von der Art der verwendeten Base. Vorzugsweise werden in dem erfindungsgemäßen Verfahren vorzugsweise von 0 Mol-% bis 100 Mol-%, bevorzugt von 0 Mol-% bis 50 Mol-%, besonders bevorzugt von 0 Mol-% bis 20 Mol-% und ganz besonders bevorzugt von 0 Mol-% bis 10 Mol-% an basischer Komponente bezogen auf das eingesetzte Olefin eingesetzt. Es ist auch möglich, eine Mischung mehrerer Basen in dem erfindungsgemäßen Verfahren einzusetzen.

Für das erfindungsgemäße Verfahren ist es vorteilhaft, wenn keine sauren Komponenten im Reaktionsgemisch vorliegen, da diese zur vermehrten Bildung von Nebenprodukten durch Isomerisierung führen. Dieses kann durch die Verwendung von neutralen Reduktionsmitteln, wie beispielsweise Wasserstoff oder Formiaten, sichergestellt werden.

Bei dem Einsatz von reiner Ameisensäure als Reduktionsmittel kann eine äquimolare Menge einer Base, wie beispielsweise Triethylamin, zugegeben werden. Bevorzugt ist ein erfindungsgemäßes Verfahren, bei dem das Reduktionsmittel Ameisensäure ohne oder mit einer unterstöchiometrischen Menge an Base, vollständig umgesetzt wird, dies bedeutet ein stöchiometrisches Verhältnis von Base zu Ameisensäure von 0:1 bis 1:1. Besonders bevorzugt ist die Ausführungsform, bei der eine Dosierung der Ameisensäure bei Reaktionstemperatur erfolgt, woraus, aufgrund der hohen Reaktionsgeschwindigkeit, eine spontane Umsetzung resultiert und sich keine Ameisensäure im Reaktionsgemisch anreichern kann. In diesem Fall ist die Menge der benötigten Base deutlich reduziert oder es kann sogar ganz auf sie verzichtet werden.

Die Hydrodimerisierung wird bei dem erfindungsgemäßen Verfahren vorzugsweise bei einem Druck von 1 bis 100 bar und bevorzugt von 6 bis 25 bar durchgeführt.

Die Temperatur während der Hydrodimerisierung beträgt in dem erfindungsgemäßen Verfahren vorzugsweise von 20 bis 160 °C, bevorzugt von 60 bis 120 °C und besonders bevorzugt von 70 bis 100°C.

Das erfindungsgemäße Verfahren kann kontinuierlich oder diskontinuierlich betrieben werden und ist nicht auf den Einsatz bestimmter Reaktortypen begrenzt. Beispiele für Reaktoren, in denen die Reaktion durchgeführt werden kann, sind Rührkesselreaktor, Rührkesselkaskade, Strömungsrohr und Schlaufenreaktor. Auch Kombinationen verschiedener Reaktoren sind möglich, beispielsweise ein Rührkesselreaktor mit nachgeschaltetem Strömungsrohr.

Die bei der Reaktion entstehende Reaktionswärme wird nach bekannten Verfahren abgeführt, beispielsweise durch interne oder externe Kühler. Konkret kann dies den Einsatz von Rohrbündelreaktoren, Reaktoren mit Kühlfingern, Kühlschlangen oder -platten oder die Kühlung eines Rückführungsstroms (Reaktoren mit Kreislauf, Recycling), bedeuten.

Für das erfindungsgemäße Verfahren ist es nicht notwendig, einen vollständigen Umsatz des nicht-cyclischen Olefins in der Hydrodimerisierungssreaktion zu erreichen. Der Umsatz des nicht-cyclischen Olefins liegt daher bevorzugt von 40 % bis 100 %, besonders bevorzugt von 60 % bis 99 %, ganz besonders bevorzugt von 85 % bis 98 %. Bei einem vollständigen Umsatz des nicht-cyclischen Olefins in dem erfindungsgemäßen Verfahrens kann es zu einer vermehrten Bildung von Nebenprodukten kommen, da das Reduktionsmittel nicht mehr vollständig abreagiert. Bei der Verwendung von Ameisensäure als Reduktionsmittel kann es hierbei zu Isomerisierungsreaktionen führen. Bei dem Einsatz von Wasserstoff als Reduktionsmittel können bei einem vollständigen Umsatz Hydrierungen beobachtet werden. Die Hydrodimerisierung in dem erfindungsgemäßen Verfahren wird vorteilhaft unter Ausschluss von Sauerstoff durchgeführt, da Sauerstoff die Polymerisation von nicht-cyclischen Olefinen mit mindestens zwei konjugierten Doppelbindungen begünstigt und dadurch die Ausbeute an substituierten oder unsubstituierten 1,7-Diolefinen herabsetzt.

Die Edukte des erfindungsgemäßen Verfahrens können alle zusammen vorgelegt und anschließend die entsprechenden Reaktionsbedingungen eingestellt werden. In einer besonderen Ausführungsform des erfindungsgemäßen Verfahrens wird zunächst der Katalysator in-situ erzeugt und anschließend erfolgt die Zugabe des Stabilisators und des nicht-cyclischen Olefins und die entsprechenden Reaktionsbedingungen werden danach eingestellt. Insbesondere empfiehlt es sich in dem erfindungsgemäßen Verfahren das Reduktionsmittel im Verlauf der Reaktion zuzudosieren und nicht vorzulegen - insbesondere beim Einsatz von Ameisensäure als Reduktionsmittel. Bereits eine zu schnelle Zudosierung der Ameisensäure als Reduktionsmittel kann zu niedrigeren Ausbeuten an 1,7-Diolefinen führen.

Die nachfolgenden Beispiele sollen das erfindungsgemäße Verfahren näher erläutern, ohne dass die Erfindung auf diese Ausführungsform beschränkt sein soll.

**Beispiel 1:** Herstellung des Carbenliganden Bis(2,4,6-trimethylphenyl)-imidazolium-o-kresolat-o-kresol

Die Herstellung des 1,3-Bis(2,4,6-trimethylphenyl)-imidazolium-chlorids erfolgt gemäß dem Verfahren beschrieben in Organometallics 1999, 18, 529 - 533. Das auf diese Weise hergestellte 1,3-Bis(2,4,6-trimethylphenyl)-imidazolium-chlorid wird in das entsprechende Kresolat wie folgt überführt: 2,35 g NaOH werden in 45 g Wasser gelöst und anschließend werden 12,7 g aufgeschmolzenes o-Kresol zugeben. Es entsteht eine milchig graue Flüssigkeit. Diese Lösung wird langsam unter Rühren zu einer Lösung von 20 g 1,3-Bis(2,4,6-trimethylphenyl)-imidazolium-chlorid in 45 g Wasser zugeben. Es fällt ein hellgrauer Feststoff aus, der mit einer G4-Fritte abfiltriert wird. Anschließend wird mit etwas Wasser und Methyl-tert.-butylether gewaschen. Nach dem Trocknen werden 18,9 g Bis(2,4,6-trimethylphenyl)-imidazolium-o-kresolat-o-kresol erhalten, dies entspricht einer Ausbeute von 91 %.

**Beispiel 2** (100 ppm Pd, 1,3-Butadien mit 4-tert.-Butylkatechol stabilisiert):

Unter Schutzgas werden 0,112 g Palladiumacetat (47,5 % Pd, Fa. Acros), 0,312 g 1,3-Bis(2,4,6-trimethylphenyl)-imidazolium-o-kresolat-o-kresol und 0,108 g Natriummethanolat (> 97 %, Fa. Merck) in 200 g frisch destilliertem N-Methylpyrrolidon (NMP, ≥ 99,5 %, Fa. Merck) gelöst und 1 Stunde bei 50°C gerührt. Diese Mischung wird in einen evakuierten 21-Autoklaven der Fa. Büchi eingesaugt und anschließend werden 240 g 1,3-Butadien (1,3-Butadien 2.6, stabilisiert mit ca. 100 ppm 4-tert.-Butylkatechol (TBC), der Fa. Oxeno Olefinchemie GmbH) bei -5°C einkondensiert. Die Mengenmessung erfolgt über das Auswiegen der Druckgasflasche. Danach wird ein Druck von 11 bar mittels Argon angelegt und der Reaktor auf 75 °C erwärmt. Innerhalb von 30 Minuten werden 101 g Ameisensäure (98 - 100 %, Fa. Riedel-de Haen) zudosiert. Der Druck wird über ein Ventil konstant bei 20 bar gehalten. Es wird eine Abgasmenge von 31,7 1 gemessen. Die Nachreaktionszeit beträgt 15 min. Nach dem Abkühlen und Entspannen des Autoklavens wird die Produktmischung per GC analysiert. Nach einer einstufigen Destillation bei 50 mbar und einer maximalen Sumpftemperatur von 80 °C erfolgt eine weitere Analyse mittels GC. Die Tabelle 1 zeigt die jeweiligen Analysenergebnisse in GC-Flächen-%.

**Tabelle 1**

| | 1,3-Butadien | 1,7-Octadien | 1,6-Octadien | 4-Vinylcyclohexen | N-Methylpyrrolidon | weitere Verbindungen |
|---|---|---|---|---|---|---|
| Reaktoraustrag | 2,1 | 69,4 | 1,6 | 0,1 | 26,7 | 0,1 |
| Destillat | 0 | 97,4 | 2,4 | 0,1 | 0 | 0,1 |
| Destillationssumpf | 0 | 6,2 | 0,3 | 0 | 92,7 | 0,8 |

Als Destillat werden 215,7 g Octadiene erhalten, dies entspricht einer isolierten Ausbeute von 89,3 %. Die Reinheit bezogen auf 1,7-Octadien beträgt 97,4 %. Der Umsatz der Ameisensäure ist vollständig.

**Beispiel 3** (50 ppm Pd, 1,3-Butadien mit 4-tert.-Butylkatechol stabilisiert):

Unter Schutzgas werden 0,056 g Palladiumacetat, 0,26 g 1,3-Bis(2,4,6-trimethylphenyl)-imidazolium-o-kresolat-o-kresol und 0,108 g Natriummethanolat in 200 g frisch destilliertem N-Methylpyrrolidon gelöst und 1 Stunde bei 50°C gerührt. Diese Mischung wird in einen evakuierten 21-Autoklaven der Fa. Büchi eingesaugt und anschließend werden 240 g 1,3-Butadien (stabilisiert mit ca. 100 ppm 4-tert.-Butylkatechol) bei -5°C einkondensiert. Die Mengenmessung erfolgt über das Auswiegen der Druckgasflasche. Danach wird ein Druck von 14 bar mittels Argon angelegt und der Reaktor auf 75 °C erwärmt. Innerhalb von 30 Minuten werden 93 g Ameisensäure zudosiert. Der Druck wird über ein Ventil konstant bei 20 bar gehalten. Es wird eine Abgasmenge von < 1 1 gemessen. Die Nachreaktionszeit beträgt 30 Minuten. Nach dem Abkühlen und Entspannen des Autoklavens wird die Produktmischung per GC analysiert. Die Tabelle 2 zeigt die jeweiligen Analysenergebnisse in GC-Flächen-%.

**Tabelle 2**

| | 1,3-Butadien | 1,7-Octadien | 1,6-Octadien | 4-Vinylcyclohexen | N-Methylpyrrolidon | weitere Verbindungen |
|---|---|---|---|---|---|---|
| Reaktoraustrag | 14,2 | 7,3 | 7,6 | 0,3 | 70,2 | 0,7 |

Es werden 318,4 g an Rohproduktgemisch erhalten. Die Restmenge an Ameisensäure beträgt 82,4 g (titrimetrisch bestimmt), d.h. der Umsatz beträgt lediglich 11 % bezogen auf die eingesetzte Ameisensäure.

**Beispiel 4** (nicht erfindungsgemäß)

Unter Schutzgas werden 0,112 g Palladiumacetat, 0,134 g 1,3-Dimethyl-imidazolium-iodid (hergestellt gemäß Beispiel 1 A in WO 97/34875) und 0,108 g Natriummethanolat in 200 g frisch destilliertem N-Methylpyrrolidon gelöst und 1 Stunde bei 50 °C gerührt. Diese Mischung wird in einem evakuierten 21-Autoklaven der Fa. Büchi eingesaugt und anschließend werden 240 g 1,3-Butadien (stabilisiert mit ca. 100 ppm 4-tert.-Butylkatechol) bei -5°C einkondensiert.

Die Mengenmessung erfolgt über das Auswiegen der Druckgasflasche. Danach wird ein Druck von 14 bar mittels Argon angelegt und der Reaktor auf 75 °C erwärmt. Innerhalb von 64 Minuten werden 101 g Ameisensäure zudosiert. Der Druck wird über ein Ventil konstant bei 20 bar gehalten. Es wird keine Abgasentwicklung gemessen. Nach dem Abkühlen und Entpannen des Autoklavens wird die Produktmischung per GC analysiert. Eine Zusammenstellung der Analyseergebnisse zeigt die Tabelle 3.

**Tabelle 3**

| | 1,3-Butadien | 1,7-Octadien | 1,6-Octadien | 4-Vinylcyclohexen | N-Methylpyrrolidon | weitere Verbindungen |
|---|---|---|---|---|---|---|
| Reaktoraustrag | 10,5 | 0 | 0 | 0,3 | 88,8 | 0,4 |

Es konnte kein 1,7-Octadien in dem Rohproduktgemisch nachgewiesen werden.

**Beispiel 5** (nicht erfindungsgemäß)

Unter Schutzgas werden 0,112 g Palladiumacetat, 0,109 g 1,3-Di-i-propyl-imidazoliumchlorid (> 97 %, Fa. ABCR) und 0,108 g Natriummethanolat in 200 g frisch destilliertem N-Methylpyrrolidon gelöst und 1 Stunde bei 50°C gerührt. Diese Mischung wird in einen evakuierten 21-Autoklaven der Fa. Büchi eingesaugt und anschließend werden 240 g 1,3-Butadien (stabilisiert mit ca. 100 ppm 4-tert.-Butylkatechol) bei -5°C einkondensiert. Die Mengenmessung erfolgt über das Auswiegen der Druckgasflasche. Danach wird ein Druck von 11 bar mittels Argon angelegt und der Reaktor auf 75 °C erwärmt. Innerhalb von 65 Minuten werden 101 g Ameisensäure zudosiert. Der Druck wird über ein Ventil konstant bei 20 bar gehalten. Es wird eine Abgasmenge von 2,9 1 gemessen. Die Nachreaktionszeit beträgt 30 Minuten. Nach dem Abkühlen und Entspannen des Autoklavens wird die Produktmischung per GC analysiert. Eine Zusammenstellung der Analyseergebnisse zeigt die Tabelle 4.

**Tabelle 4**

| | 1,3-Butadien | 1,7-Octadien | 1,6-Octadien | 4-Vinylcyclohexen | N-Methylpyrrolidon | weitere Verbindungen |
|---|---|---|---|---|---|---|
| Reaktoraustrag | 14,4 | 0 | 0 | 0,5 | 85,1 | 0 |

Es konnte kein 1,7-Octadien in dem Rohproduktgemisch nachgewiesen werden.

**Beispiel 6** (nicht erfindungsgemäß)

Unter Schutzgas werden 0,112 g Palladiumacetat, 0,130 g 1,3-Di-tert.-butyl-imidazolium-chlorid (> 97 %, Fa. ABCR) und 0,108 g Natriummethanolat in 200 g frisch destilliertem N-Methylpyrrolidon gelöst und 1 Stunde bei 50°C gerührt. Diese Mischung wird in einen evakuierten 21-Autoklaven der Fa. Büchi eingesaugt und anschließend werden 240 g 1,3-Butadien (stabilisiert mit ca. 100 ppm 4-tert.-Butylkatechol) bei -5°C einkondensiert. Die Mengenmessung

erfolgt über das Auswiegen der Druckgasflasche. Danach wird ein Druck von 11 bar mittels Argon angelegt und der Reaktor auf 75°C erwärmt. Innerhalb von 60 Minuten werden 101 g Ameisensäure zudosiert. Der Druck wird über ein Ventil konstant bei 20 bar gehalten. Es wird eine Abgasmenge von 2,8 1 gemessen. Die Nachreaktionszeit beträgt 30 Minuten. Nach dem Abkühlen und Entspannen des Autoklavens wird die Produktmischung per GC analysiert. Eine Zusammenstellung der Analyseergebnisse zeigt die Tabelle 5.

**Tabelle 5**

| | 1,3-Butadien | 1,7-Octadien | 1,6-Octadien | 4-Vinylcyclohexen | N-Methylpyrrolidon | weitere Verbindungen |
|---|---|---|---|---|---|---|
| Reaktoraustrag | 10 | 0 | 0 | 0,4 | 89,2 | 0,4 |

Es konnte kein 1,7-Octadien in dem Rohproduktgemisch nachgewiesen werden.

**Beispiel 7** (nicht erfindungsgemäß)

Unter Schutzgas werden 0,112 g Palladiumacetat, 0,109 g 1,3-Di-tert.-butyl-imidazolium-2-yliden (98 %, Fa. Strem) und 0,108 g Natriummethanolat in 200 g frisch destilliertem N-Methylpyrrolidon gelöst und 1 Stunde bei 50°C gerührt. Diese Mischung wird in einen evakuierten 21-Autoklaven der Fa. Büchi eingesaugt und anschließend werden 240 g 1,3-Butadien (stabilisiert mit ca. 100 ppm 4-tert.-Butylkatechol) bei -5°C einkondensiert. Die Mengenmessung erfolgt über das Auswiegen der Druckgasflasche. Danach wird ein Druck von 14 bar mittels Argon angelegt und der Reaktor auf 75°C erwärmt. Innerhalb von 66 Minuten werden 101 g Ameisensäure zudosiert. Der Druck wird über ein Ventil konstant bei 20 bar gehalten. Es wird eine Abgasmenge von 3,42 1 gemessen. Die Nachreaktionszeit beträgt 30 Minuten. Nach dem Abkühlen und Entspannen des Autoklavens wird die Produktmischung per GC analysiert. Eine Zusammenstellung der Analyseergebnisse zeigt die Tabelle 6.

**Tabelle 6**

| | 1,3-Butadien | 1,7-Octadien | 1,6-Octadien | 4-Vinylcyclohexen | N-Methylpyrrolidon | weitere Verbindungen |
|---|---|---|---|---|---|---|
| Reaktoraustrag | 11,7 | 0,2 | 0 | 0,5 | 87,1 | 0,5 |

Es konnten lediglich 0,2 GC-Flächen-% an 1,7-Octadien in dem Rohproduktgemisch nachgewiesen werden.

Die Beispiele 4 - 7 zeigen deutlich, dass nicht alle Metall-Carben-Komplexe eine Hydrodimerisierung von 1,3-Butadien zum 1,7-Octadien katalysieren.

**Beispiel 8** (30 ppm Pd, 1,3-Butadien stabilisiert mit 100 ppm 4-Hydroxy-2,2,6,6-tetramethylpiperidin-N-oxyl bezogen auf 1,3-Butadien):

Unter Schutzgas werden 0,056 g Palladiumacetat, 0,521 g 1,3-Bis(2,4,6-trimethylphenyl)-imidazolium-o-kresolat-o-kresol und 0,27 g Natriummethanolat in 200 g frisch destilliertem N-Methylpyrrolidon gelöst, 1 Stunde bei 50°C gerührt und danach auf 20°C abgekühlt. Anschließend werden 0,32 g 4-Hydroxy-2,2,6,6-tetramethylpiperidin-N-oxyl (SiYPro C710 der Fa. Degussa AG) zugegeben. Diese Mischung wird in einem evakuierten 21-Autoklaven der Fa. Büchi eingesaugt und anschließend werden 480 g 1,3-Butadien (unstabilisiert) bei-5°C einkondensiert. Die Mengenmessung erfolgt über das Auswiegen der Druckgasflasche. Danach wird ein Druck von 10 bar mittels Argon angelegt und der Reaktor auf 75 °C erwärmt. Innerhalb von 120 Minuten werden 202 g Ameisensäure (98 - 100 %) zudosiert. Die Temperatur wird nach Anspringen der Reaktion zwischen 80°C und 90°C gehalten. Der Druck wird über ein Ventil konstant bei 20 bar gehalten. Es wird eine Abgasmenge von 82,7 1 gemessen. Die Nachreaktionszeit beträgt 30 Minuten. Nach dem Abkühlen und Entspannen des Autoklavens wird die Produktmischung per GC analysiert. Nach einer einstufigen Destillation bei 50 mbar und einer maximalen Sumpftemperatur von 80 °C erfolgt eine weitere Analyse mittels GC. Die Tabelle 7 zeigt die jeweiligen Analysenergebnisse in GC-Flächen-%.

**Tabelle 7**

| | 1,3-Butadien | 1,7-Octadien | 1,6-Octadien | 4-Vinylcyclohexen | N-Methylpyrrolidon | weitere Verbindungen |
|---|---|---|---|---|---|---|
| Reaktoraustrag | 7,6 | 70,0 | 4,2 | 0,2 | 17,8 | 0,2 |
| Destillat | 0 | 94,3 | 5,5 | 0,2 | 0 | 0 |
| Destillationssumpf | 0 | 6,2 | 0,8 | 0,1 | 90,8 | 2,1 |

Als Destillat werden 336,3 g Octadiene erhalten, dies entspricht einer isolierten Ausbeute von 69,4 %. Die Reinheit bezogen auf 1,7-Octadien beträgt 94,3 %. Der Umsatz der Ameisensäure ist vollständig.

**Beispiel 9** (28 ppm Pd, Base, 1,3-Butadien stabilisiert mit 100 ppm 4-Hydroxy-2,2,6,6-tetramethylpiperidin-N-oxyl bezogen auf 1,3-Butadien):

Unter Schutzgas werden 0,056 g Palladiumacetat, 0,521 g 1,3-Bis(2,4,6-trimethylphenyl)-imidazolium-o-kresolat-o-kresol und 0,27 g Natriummethanolat in 200 g frisch destilliertem N-Methylpyrrolidon gelöst, 1 Stunde bei 50°C gerührt und danach auf 20°C abgekühlt. Anschließend werden 0,32 g 4-Hydroxy-2,2,6,6-tetramethylpiperidin-N-oxyl (SiYPro C710 der Fa. Degussa AG) und 44,4 g Triethylamin als Base zugegeben. Diese Mischung wird in einem evakuierten 21-Autoklaven der Fa. Büchi eingesaugt und anschließend werden 480 g 1,3-Butadien (unstabilisiert) bei -5°C einkondensiert. Die Mengenmessung erfolgt über das Auswiegen der Druckgasflasche. Danach wird ein Druck von 10 bar mittels Argon angelegt und der Reaktor auf 75 °C erwärmt. Innerhalb von 123 Minuten werden 202 g Ameisensäure (98 - 100 %) zudosiert. Die Temperatur wird nach Anspringen der Reaktion zwischen 80°C und 90°C gehalten. Der Druck wird über ein Ventil konstant bei 20 bar gehalten. Es wird eine Abgasmenge von 77 1 gemessen. Die Nachreaktionszeit beträgt 15 min. Nach dem Abkühlen und Entspannen des Autoklavens wird die Produktmischung per GC analysiert. Nach einer Destillation und der Abnahme von zwei Fraktionen bis 50 mbar und einer maximalen Sumpftemperatur von 80 °C erfolgt eine weitere Analyse mittels GC. Die Tabelle 8 zeigt die jeweiligen Analysenergebnisse in GC-Flächen-%.

**Tabelle 8**

| | 1,3-Butadien | Triethylamin | 1,7-Octadien | 1,6-Octadien | 4-Vinylcyclohexen | N-Methylpyrrolidon | weitere Verbindungen |
|---|---|---|---|---|---|---|---|
| Reaktoraustrag | 2,8 | 5,6 | 69,4 | 4,9 | 0,2 | 16,6 | 0,5 |
| Destillat 1 | 0 | 27,4 | 69,3 | 3,2 | 0,1 | 0 | 0 |
| Destillat 2 | 0 | 2,9 | 90,1 | 6,5 | 0,2 | 0,1 | 0,2 |
| Destillationssumpf | 0 | 0,7 | 5,6 | 1,0 | 0,1 | 89,5 | 3,1 |

In den Destillaten werden 400,8 g Octadiene erhalten, dies entspricht einer Ausbeute von 82,7 %. Die Reinheit der Octadiene bezogen auf 1,7-Octadien beträgt 93,5 %. Der Umsatz der Ameisensäure ist vollständig.

**Beispiel 10** (15 ppm Pd, 1,3-Butadien stabilisiert mit 100 ppm 4-Hydroxy-2,2,6,6-tetramethylpiperidin-N-oxyl bezogen auf 1,3-Butadien):

Unter Schutzgas werden 0,028 g Palladiumacetat, 0,26 g 1,3-Bis(2,4,6-trimethylphenyl)-imidazolium-o-kresolat-o-kresol und 0,13 g Natriummethanolat in 200 g frisch destilliertem N-Methylpyrrolidon gelöst, 1 Stunde bei 50°C gerührt und danach auf 20°C abgekühlt. Anschließend werden 0,32 g 4-Hydroxy-2,2,6,6-tetramethylpiperidin-N-oxyl (SiYPro C710 der Fa. Degussa AG) zugegeben. Diese Mischung wird in einem evakuierten 21-Autoklaven der Fa. Büchi eingesaugt und anschließend werden 480 g 1,3-Butadien (unstabilisiert) bei - 5°C einkondensiert. Die Mengenmessung erfolgt über das Auswiegen der Druckgasflasche. Danach wird ein Druck von 10 bar mittels Argon angelegt und der Reaktor auf 80 °C erwärmt. Innerhalb von 240 Minuten werden 193 g Ameisensäure (98 - 100 %) zudosiert. Die Temperatur wird nach Anspringen der Reaktion mit Hilfe einer innen liegenden Kühlschlange zwischen 80°C und 81°C gehalten. Der Druck wird über ein Ventil konstant bei 20 bar gehalten. Es wird eine Abgasmenge von 71 1 gemessen. Die Nachreaktionszeit beträgt 10 Minuten. Nach dem Abkühlen und Entspannen des Autoklavens wird die Produktmischung per GC analysiert. Nach einer einstufigen Destillation bei 100 - 50 mbar und einer maximalen Sumpftemperatur von 80 °C erfolgt eine weitere Analyse mittels GC. Die Tabelle 9 zeigt die jeweiligen Analysenergebnisse in GC-Flächen-%.

**Tabelle 9**

| | 1,3-Butadien | 1,7-Octadien | 1,6-Octadien | 4-Vinylcyclohexen | N-Methylpyrrolidon | weitere Verbindungen |
|---|---|---|---|---|---|---|
| Reaktoraustrag | 1,8 | 77,0 | 2,6 | 0,3 | 18,2 | 0,1 |
| Destillat | 0 | 96,1 | 3,4 | 0,3 | 0,2 | 0 |
| Destillationssumpf | 0 | 6,4 | 0,5 | 0,1 | 91,9 | 1,1 |

Als Destillat werden 421,2 g Octadiene erhalten, dies entspricht einer isolierten Ausbeute von 94,7 %. Die Reinheit bezogen auf 1,7-Octadien beträgt 96,1 %. Der Umsatz der Ameisensäure ist vollständig.

**Beispiel 11** (6 ppm Pd, 1,3-Butadien stabilisiert mit 100 ppm 4-Hydroxy-2,2,6,6-tetramethylpiperidin-N-oxyl bezogen auf 1,3-Butadien):

Unter Schutzgas werden 0,0112 g Palladiumacetat, 0,521 g 1,3-Bis(2,4,6-trimethylphenyl)-imidazolium-o-kresolat-o-kresol und 0,13 g Natriummethanolat in 200 g frisch destilliertem N-Methylpyrrolidon gelöst, 1 Stunde bei 50°C gerührt und danach auf 20°C abgekühlt. Anschließend werden 0,32 g 4-Hydroxy-2,2,6,6-tetramethylpiperidin-N-oxyl (SiYPro C710 der Fa. Degussa AG) zugegeben. Diese Mischung wird in einem evakuierten 21-Autoklaven der Fa. Büchi eingesaugt und anschließend werden 480 g 1,3-Butadien (unstabilisiert) bei - 5°C einkondensiert. Die Mengenmessung erfolgt über das Auswiegen der Druckgasflasche. Danach wird ein Druck von 10 bar mittels Argon angelegt und der Reaktor auf 80 °C erwärmt. Innerhalb von 12 Stunden werden 193 g Ameisensäure (98 - 100 %) zudosiert. Die Temperatur wird nach Anspringen der Reaktion mit Hilfe einer innen liegenden Kühlschlange zwischen 80°C und 81°C gehalten. Der Druck wird über ein Ventil konstant bei 20 bar gehalten. Es wird eine Abgasmenge von 71,4 1 gemessen. Die Nachreaktionszeit beträgt 10 Minuten. Nach dem Abkühlen und Entspannen des Autoklavens wird die Produktmischung per GC analysiert. Nach einer einstufigen Destillation bei 100 - 50 mbar und einer maximalen Sumpftemperatur von 80 °C erfolgt eine weitere Analyse mittels GC. Die Tabelle 10 zeigt die jeweiligen Analysenergebnisse in GC-Flächen-%.

**Tabelle 10**

| | 1,3-Butadien | 1,7-Octadien | 1,6-Octadien | 4-Vinylcyclohexen | N-Methylpyrrolidon | weitere Verbindungen |
|---|---|---|---|---|---|---|
| Reaktoraustrag | 1,7 | 77,6 | 2,5 | 0,6 | 17,6 | 0 |
| Destillat | 0 | 96,1 | 3,1 | 0,7 | 0 | 0,1 |
| Destillationssumpf | 0 | 6,1 | 0,2 | 0,3 | 92,1 | 1,3 |

Als Destillat werden 395,6 g Octadiene erhalten, dies entspricht einer isolierten Ausbeute von 85,5 %. Hieraus ergibt sich eine Katalysatorproduktivität TON (Octadiene) = 72248. Die Reinheit bezogen auf 1,7-Octadien beträgt 96,1 %. Der Umsatz der Ameisensäure ist vollständig. Hervorzuheben ist, dass selbst bei langen Reaktionszeiten bei diesem Herstellverfahren keine weitere Isomerisierung des 1,7-Octadiens auftritt.

Die Beispiele 8 und 11 zeigen deutlich, dass bei Verwendung von N-Oxylradikalen als Stabilisatoren für 1,3-Butadien, wie beispielsweise 4-Hydroxy-2,2,6,6,-tetramethylpiperidin-N-oxyl, auch geringe Mengen an Katalysator von < 50 ppm ausreichend sind. Im Gegensatz hierzu zeigt das Beispiel 3 unter Verwendung des Stabilisators 4-tert.-Butylkatechol für 1,3-Butadien und einer Katalysatormenge von 50 ppm eine deutlich schlechtere Ausbeute.

**Beispiel 12** (45 ppm Pd, Isopren stabilisiert mit 100 ppm 4-Hydroxy-2,2,6,6-tetramethylpiperidin-N-oxyl bezogen auf 1,3-Butadien):

Unter Schutzgas werden 0,112 g Palladiumacetat, 0,521 g 1,3-Bis(2,4,6-trimethylphenyl)-imidazolium-o-kresolat-o-kresol und 0,27 g Natriummethanolat in 200 g frisch destilliertem N-Methylpyrrolidon gelöst, 1 Stunde bei 50°C gerührt und danach auf 20°C abgekühlt. Anschließend werden 0,32 g 4-Hydroxy-2,2,6,6-tetramethylpiperidin-N-oxyl (SiYPro C710 der Fa. Degussa AG) zugegeben. Diese Mischung wird in einen evakuierten 21-Autoklaven der Fa. Büchi eingesaugt und anschließend werden 606 g frisch destilliertes Isopren (unstabilisiert) eingesaugt. Danach wird ein Druck von 14 bar mittels Argon angelegt und der Reaktor auf 80 °C erwärmt. Innerhalb von 240 Minuten werden 193 g Ameisensäure (98 - 100 %) zudosiert. Die Temperatur wird nach Anspringen der Reaktion mit Hilfe einer innen liegenden

Kühlschlange zwischen 80°C und 81°C gehalten. Der Druck wird über ein Ventil konstant bei 20 bar gehalten. Es wird eine Abgasmenge von 73,4 1 gemessen. Die Nachreaktionszeit beträgt 20 Minuten. Nach dem Abkühlen und Entspannen des Autoklavens wird die Produktmischung per GC analysiert. Nach einer einstufigen Destillation bei 100 - 40 mbar und einer maximalen Sumpftemperatur von 105 °C erfolgt eine weitere Analyse mittels GC. Die Tabelle 11 zeigt die jeweiligen Analysenergebnisse in GC-Flächen-%.

**Tabelle 11**

| | Isopren | Dimethyloctadien 1 (Isomere 1) | Dimethyloctadien 2 (Isomere 2) | Dimethyloctadien 3 (Isomere 3) | N-Methylpyrrolidon | weitere Verbindungen |
|---|---|---|---|---|---|---|
| Reaktoraustrag | 11,1 | 40,1 | 30,9 | 4,1 | 12,7 | 1,1 |
| Destillat | 0 | 51,9 | 40,1 | 5,0 | 1,1 | 1,9 |
| Destillationssumpf | 0 | 0,2 | 1,5 | 1,3 | 94,8 | 2,2 |

Als Destillat werden 510,8 g isomerer Dimethyloctadiene erhalten, dies entspricht einer isolierten Ausbeute von 83,2 %.

**Beispiel 13** (57 ppm Pd, Isopren und 1,3-Butadien stabilisiert mit 100 ppm 4-Hydroxy-2,2,6,6-tetramethylpiperidin-N-oxyl bezogen auf 1,3-Butadien):

Unter Schutzgas werden 0,112 g Palladiumacetat, 0,521 g 1,3-Bis(2,4,6-trimethylphenyl)-imidazolium-o-kresolat-o-kresol und 0,27 g Natriummethanolat in 200 g frisch destilliertem N-Methylpyrrolidon gelöst, 1 Stunde bei 50°C gerührt und danach auf 20°C abgekühlt. Anschließend werden 0,32 g 4-Hydroxy-2,2,6,6-tetramethylpiperidin-N-oxyl (SiYPro C710 der Fa. Degussa AG) zugegeben. Diese Mischung wird in einem evakuierten 21-Autoklaven der Fa. Büchi eingesaugt und anschließend werden 303 g frisch destilliertes Isopren (unstabilisiert) eingesaugt. Danach werden 240 g 1,3-Butadien (unstabilisiert) bei -5°C einkondensiert. Dann wird ein Druck von 14 bar mittels Argon angelegt und der Reaktor auf 80 °C erwärmt. Innerhalb von 60 Minuten werden 193 g Ameisensäure (98 - 100 %) zudosiert. Die Temperatur wird nach Anspringen der Reaktion mit Hilfe einer innen liegenden Kühlschlange zwischen 80°C und 81°C gehalten. Der Druck wird über ein Ventil konstant bei 20 bar gehalten. Es wird eine Abgasmenge von 79,2 1 gemessen. Die Nachreaktionszeit beträgt 20 Minuten. Nach dem Abkühlen und Entspannen des Autoklavens wird die Produktmischung per GC analysiert. Nach einer einstufigen Destillation bei 100 - 40 mbar und einer maximalen Sumpftemperatur von 105 °C erfolgt eine weitere Analyse mittels GC. Die Tabelle 12 zeigt die jeweiligen Analysenergebnisse in GC-Flächen-%.

**Tabelle 12**

| | Isopren / 1,3-Butadien | Octadien | Methyloctadien | Dimethyloctadien | N-Methylpyrrolidon | weitere Verbindungen |
|---|---|---|---|---|---|---|
| Reaktoraustrag | 5,5 | 21,4 | 22,4 | 31,0 | 15,5 | 4,2 |
| Destillat | 0,1 | 26,5 | 27,6 | 39,4 | 0,8 | 5,6 |
| Destillationssumpf | 0 | 0 | 0 | 0 | 97,6 | 2,4 |

Als Destillat wird eine Mischung von isomeren C8 - C10 Diolefinen erhalten. Hierbei handelt es sich hauptsächlich um Octadien, Methyloctadien und Dimethyloctadien. Es werden 441,9 g als Destillat erhalten, dies entspricht einer isolierten Ausbeute von 88,4 % an Hydrodimerisierungsprodukten.

**Beispiel 14** (nicht erfindungsgemäß; 30 ppm Pd, 1,3-Butadien stabilisiert mit 100 ppm 4-Hydroxy-2,2,6,6-tetramethylpiperidin-N-oxyl bezogen auf 1,3-Butadien):

Unter Schutzgas werden 0,056 g Palladiumacetat, 0,521 g 1,3-Bis(2,4,6-trimethylphenyl)-imidazolium-o-kresolat-o-kresol und 0,27 g Natriummethanolat in 200 g frisch destilliertem N-Methylpyrrolidon gelöst, 1 Stunde bei 50°C gerührt und danach auf 20°C abgekühlt. Anschließend werden 0,32 g 4-Hydroxy-2,2,6,6-tetramethylpiperidin-N-oxyl (SiYPro C710 der Fa. Degussa AG) zugegeben. Diese Mischung wird in einen evakuierten 21-Autoklaven der Fa. Büchi eingesaugt und anschließend werden 480 g frisch destilliertes 1,3-Butadien (unstabilisiert) bei -5°C einkondensiert. Die Mengenmessung erfolgt über das Auswiegen der Druckgasflasche. Danach werden 202 g Ameisensäure (98 - 100 %) mit einer Pumpe zudosiert. Anschließend wird ein Druck von 10 bar mittels Argon angelegt und der Reaktor auf 75 °C erwärmt und 2 Stunden lang gerührt. Der Druck wird über ein Ventil konstant bei 20 bar gehalten. Während dieser Zeit wird keine Abgasentwicklung beobachtet. Die Nachreaktionszeit beträgt 30 Minuten. Nach dem Abkühlen und Entspannen des Autoklavens wird die Produktmischung per GC analysiert. Es wird kein 1,7-Octadien in der Reaktionsmischung nachgewiesen.

## Patentansprüche

1. Verfahren zur Herstellung von substituierten oder unsubstituierten 1,7-Diolefinen durch Hydrodimerisierung von nicht-cyclischen Olefinen mit mindestens zwei konjugierten Doppelbindungen in Gegenwart eines Reduktionsmittels und eines Katalysators,
**dadurch gekennzeichnet,**
**dass** als Katalysator ein Metall-Carben-Komplex eingesetzt wird, der ein Metall der 8. bis 10. Gruppe des Periodensystems und mindestens einen Carben-Liganden gemäß der Struktur **1, 2, 3** oder **4**

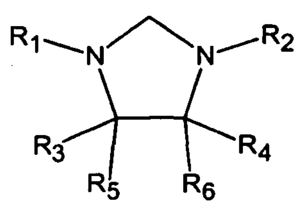

**(1)**

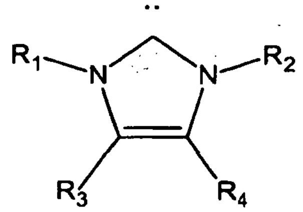

**(2)**

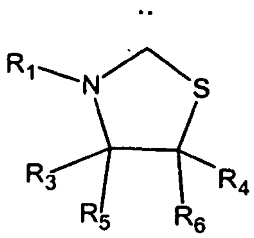

**(3)**

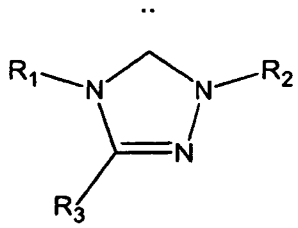

**(4)**

mit:

$R_1$, $R_2$ =-$(CH_2)_n$-B
B = mono- oder polycyclische Arylgruppe mit 6 bis 14 Kohlenstoffatomen oder mono- oder polycyclischer Heterocyclus mit 5 bis 14 Kohlenstoff- und Heteroatomen, wobei diese Heterocyclus 1 bis 3 Heteroatome, ausgewählt aus der Gruppe N, O und S, aufweist,
n = 0 - 4
$R_3$, $R_4$, $R_5$ und $R_6$ = Wasserstoff, Alkyl-, Heteroaryl-, Aryl-, -CN, -COOH, -COO-Alkyl-, -COO-Aryl-, -OCO-Alkyl-, OCO-Aryl-, -OCOO-Alkyl-, OCOO-Aryl-, -CHO, -CO-Alkyl-, -CO-Aryl-, $-NH_2$, -NH(Alkyl)-, $-N(Alkyl)_2$-, NH(Aryl)-, $-N(Aryl)_2$-, -F, - Cl, -Br, -I, -OH, $-CF_3$, $-NO_2$, -Ferrocenyl, $-SO_3H$, $-PO_3H_2$, wobei die Alkylgruppen von 1 bis 12 und die Arylgruppen von 5 bis 14 Kohlenstoffatome aufweisen und die Substituenten vom Typ $R_3$ und $R_4$ auch Teil eines verbrückenden aliphatischen oder aromatischen Ringes sein können,

aufweist und die Substituenten vom Typ $R_1$ und $R_2$ gleich oder verschieden sowie substituiert oder unsubstituiert sind, und die Substituenten vom Typ $R_3$, $R_4$, $R_5$ und $R_6$ ebenfalls gleich oder verschieden sowie substituiert oder unsubstituiert sind und als Reduktionsmittel Ameisensäure und/oder Formiate eingesetzt wird.

2. Verfahren gemäß Anspruch 1,
**dadurch gekennzeichnet,**
**dass** ein Metall-Carben-Komplex eingesetzt wird, der ein Metall, ausgewählt aus Nickel, Rhodium, Palladium und/ oder Platin, aufweist.

3. Verfahren gemäß Anspruch 2,
**dadurch gekennzeichnet,**
**dass** ein Metall-Carben-Komplex eingesetzt wird, der Palladium als Metall aufweist.

4. Verfahren gemäß zumindest einem der Ansprüche 1 bis 3,
**dadurch gekennzeichnet,**
**dass** ein Metall-Carben-Komplex eingesetzt wird, der zumindest ein Carben-Liganden, ausgewählt aus den Strukturen **1** bis **4** mit $R_3$ bis $R_6$ = Wasserstoff, aufweist.

5. Verfahren gemäß Anspruch 4,
**dadurch gekennzeichnet,**
**dass** ein Metall-Carben-Komplex eingesetzt wird, der zumindest ein Carben-Liganden gemäß der Struktur **2** mit $R_3$ und $R_4$ gleich Wasserstoff aufweist.

6. Verfahren gemäß Anspruch 5,
**dadurch gekennzeichnet,**
**dass** ein Metall-Carben-Komplex eingesetzt wird, der zumindest ein Carben-Liganden gemäß der Struktur 2 mit $R_3$ und $R_4$ gleich Wasserstoff und $R_1$ und $R_2$ mit n = 0 und B gleich einer Phenyl- oder 2,4,6-Trimethylphenylgruppe, aufweist.

7. Verfahren gemäß zumindest einem der Ansprüche 1 bis 6,
**dadurch gekennzeichnet,**
**dass** die Konzentration des Katalysators, formal angegeben in ppm (Masse) an Metall bezogen auf die Gesamtmasse der Reaktionsmischung, zu Beginn der Hydrodimerisierung 1 bis 500 ppm beträgt.

8. Verfahren gemäß zumindest einem der Ansprüche 1 bis 7,
**dadurch gekennzeichnet,**
**dass** als nicht-cyclische Olefine mit mindestens zwei konjugierten Doppelbindungen Verbindungen, ausgewählt aus 1,3-Butadien, Isopren, 1,3-Pentadien, Chloropren, oder entsprechende Mischungen, die nicht-cyclische Olefine mit mindestens zwei konjugierten Doppelbindungen aufweisen, eingesetzt werden.

9. Verfahren gemäß zumindest einem der Ansprüche 1 bis 8,
**dadurch gekennzeichnet,**
**dass** nicht-cyclische Olefine eingesetzt werden, die entweder keinen Stabilisator oder einen Stabilisator, ausgewählt aus alkylierten Phenolen oder stabilen N-Oxylradikalen, aufweisen.

10. Verfahren gemäß Anspruch 9,
**dadurch gekennzeichnet,**
**dass** als Stabilisator radikalisch wirkende Stabilisatoren, ausgewählt aus alkylierten Phenolen oder stabilen N-Oxylradikale, eingesetzt werden.

11. Verfahren gemäß Anspruch 10,
**dadurch gekennzeichnet,**
**dass** N-Oxylradikale als Stabilisator eingesetzt werden, ausgewählt aus an 4-Position substituierte oder unsubstituierte 2,2,6,6-Tetramethylpiperidin-N-oxyle, 4-Hydroxy-2,2,6,6,-tetramethylpiperidin-N-oxyl oder 2,2,6,6-Tetramethylpiperidin-N-oxyl.

12. Verfahren gemäß Anspruch 8,
**dadurch gekennzeichnet,**
**dass** 1,3-Butadien als nicht-cyclisches Olefin eingesetzt wird.

13. Verfahren gemäß zumindest einem der Ansprüche 1 bis 12,
**dadurch gekennzeichnet,**
**dass** Ameisensäure als Reduktionsmittel eingesetzt wird.

14. Verfahren gemäß Anspruch 13,
**dadurch gekennzeichnet,**
**dass** die Ameisensäure im Verlauf der Reaktion zudosiert wird.

15. Verfahren gemäß zumindest einem der Ansprüche 1 bis 14,
**dadurch gekennzeichnet,**
**dass** die Hydrodimerisierung in Gegenwart von Basen durchgeführt wird.

16. Verfahren gemäß Anspruch 15,
**dadurch gekennzeichnet,**
**dass** Alkalimetallhydoxide, Alkalimetallalkoholate oder tertiäre Amine als Basen eingesetzt werden.

## Claims

1. A process for preparing substituted or unsubstituted 1,7-diolefins by hydrodimerizing non-cyclic olefins having at least two conjugated double bonds in the presence of a reducing agent and of a catalyst,
**characterized in that**
the catalyst used is a metal-carbene complex which has a metal of group 8 to 10 of the Periodic Table and at least one carbene ligand of the structure **1, 2, 3** or **4**

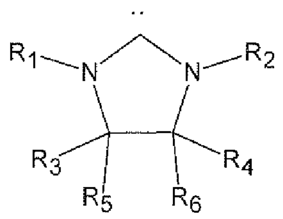
(1)

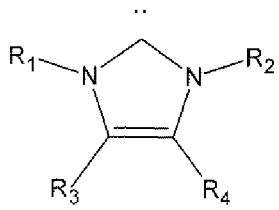
(2)

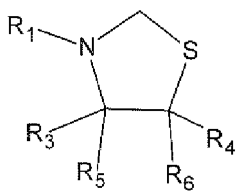
(3)

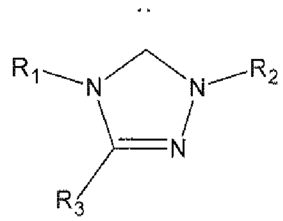
(4)

where:

$R_1$, $R_2$ = $-(CH_2)_n-B$ B = mono- or polycyclic aryl group having 6 to 14 carbon atoms or mono- or polycyclic heterocycle having 5 to 14 carbon atoms and heteroatoms, where this heterocycle has 1 to 3 heteroatoms selected from the group of N, O and S,
n = 0-4
$R_3$, $R_4$, $R_5$ and $R_6$ = hydrogen, alkyl, heteroaryl, aryl, -CN, -COOH, -COO-alkyl, -COO-aryl, -OCO-alkyl, OCO-aryl, -OCOO-alkyl, OCOO-aryl, -CHO, -CO-alkyl, -CO-aryl, $-NH_2$, -NH(alkyl), $-N(alkyl)_2$, NH(aryl), $-N(aryl)_2$, -F, -Cl, -Br, -I, -OH, $-CF_3$, $-NO_2$, -ferrocenyl, $-SO_3H$, $-PO_3H_2$, where the alkyl groups have 1 to 12 carbon atoms and the aryl groups 5 to 14 carbon atoms, and the substituents of the $R_3$ and $R_4$ type may also be part of a bridging aliphatic or aromatic ring,

and the substituents of the $R_1$ and $R_2$ type are the same or different and are substituted or unsubstituted, and the substituents of the $R_3$, $R_4$, $R_5$ and $R_6$ type are likewise the same or different and are substituted or unsubstituted and the reducing agents used are formic acid and/or formates.

**2.** A process according to claim 1,
**characterized in that**
a metal-carbene complex which has a metal selected from nickel, rhodium, palladium and/or platinum is used.

**3.** A process according to claim 2,
**characterized in that**
a metal-carbene complex which has palladium as the metal is used.

**4.** A process according to at least one of claims 1 to 3,
**characterized in that**
a metal-carbene complex which has at least one carbene ligand selected from the structures **1** to **4** where $R_3$ to $R_6$ = hydrogen is used.

**5.** A process according to claim 4,
**characterized in that**
a metal-carbene complex which has at least one carbene ligand of the structure **2** where $R_3$ and $R_4$ are each hydrogen is used.

**6.** A process according to claim 5,
**characterized in that**
a metal-carbene complex which has at least one carbene ligand of the structure **2** where $R_3$ and $R_4$ are each hydrogen and $R_1$ and $R_2$, where n = 0, and B are each a phenyl or 2,4,6-trimethylphenyl group is used.

**7.** A process according to at least one of claims 1 to 6,

**characterized in that**
the concentration of the catalyst, reported in a formal sense in ppm (mass) of metal based on the overall mass of the reaction mixture, at the start of the hydrodimerization is 1 to 500 ppm.

**8.** A process according to at least one of claims 1 to 7,
**characterized in that**
the non-cyclic olefins having at least two conjugated double bonds used are compounds selected from 1,3-butadiene, isoprene, 1,3-pentadiene, chloroprene, or corresponding mixtures which comprise non-cyclic olefins with at least two conjugated double bonds.

**9.** A process according to at least one of claims 1 to 8,
**characterized in that**
non-cyclic olefins which either have no stabilizer or have a stabilizer selected from alkylated phenols or stable N-oxyl radicals are used.

**10.** A process according to claim 9,
**characterized in that**
the stabilizers used are stabilizers which act by a free-radical mechanism, selected from alkylated phenols or stable N-oxyl radicals.

**11.** A process according to claim 10,
**characterized in that**
N-oxyl radicals selected from 4-substituted or unsubstituted 2,2,6,6-tetramethylpiperidine N-oxyls, 4-hydroxy-2,2,6,6-tetramethylpiperidine N-oxyl or 2,2,6,6-tetramethylpiperidine N-oxyl are used as stabilizers.

**12.** A process according to claim 8,
**characterized in that**
1,3-butadiene is used as the non-cyclic olefin.

**13.** A process according to at least one of claims 1 to 12,
**characterized in that**
formic acid is used as the reducing agent.

**14.** A process according to claim 13,
**characterized in that**
the formic acid is metered in in the course of the reaction.

**15.** A process according to at least one of claims 1 to 14,
**characterized in that**
the hydrodimerization is performed in the presence of bases.

**16.** A process according to claim 15,
**characterized in that**
alkali metal hydroxides, alkali metal alkoxides or tertiary amines are used as bases.

## Revendications

**1.** Procédé de fabrication de 1,7-dioléfines substituées ou non substituées par hydrodimérisation d'oléfines non cycliques contenant au moins deux doubles liaisons conjuguées en présence d'un réducteur et d'un catalyseur, **caractérisé en ce qu'**un complexe métal-carbène est utilisé en tant que catalyseur, qui comprend un métal des groupes 8 à 10 du tableau périodique et au moins un ligand carbène de structure 1, 2, 3 ou 4

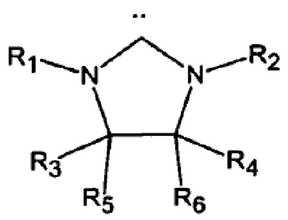
(1)

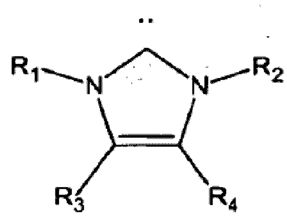
(2)

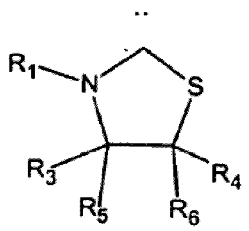
(3)

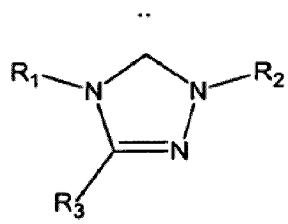
(4)

avec :

$R_1$, $R_2$ = $-(CH_2)_n-B$
B = groupe aryle mono- ou polycyclique contenant 6 à 14 atomes de carbone ou hétérocycle mono- ou polycyclique contenant 5 à 14 atomes de carbone et
hétéroatomes, cet hétérocycle comprenant 1 à 3 hétéroatomes choisis dans le groupe constitué par N, O et S,
n = 0 à 4,
$R_3$, $R_4$, $R_5$ et $R_6$ = hydrogène, alkyle, hétéroaryle, aryle, -CN, -COOH, -COO-alkyle, -COO-aryle, -OCO-alkyle, OCO-aryle, -OCOO-alkyle, O-COO-aryle, -CHO, -CO-alkyle, - CO-aryle, $-NH_2$, -NH(alkyle), $-N(alkyle)_2$, -NH (aryle), - $N(aryle)_2$, -F, -Cl, -Br, -I, -OH, $-CF_3$, $-NO_2$, - ferrocényle, $-SO_3H$, $-PO_3H_2$, les groupes alkyle comprenant 1 à 12 et les groupes aryle 5 à 14 atomes de carbone, et les substituants de type $R_3$ et $R_4$ pouvant également faire partie d'un cycle aliphatique ou aromatique ponté,
les substituants de type $R_1$ et $R_2$ étant identiques ou différents et substitués ou non substitués, et les substituants de type $R_3$, $R_4$, $R_5$ et $R_6$ étant également identiques ou différents et substitués ou non substitués, et de l'acide formique et/ou un formiate étant utilisé en tant que réducteur.

2. Procédé selon la revendication 1, **caractérisé en ce qu'**un complexe métal-carbène comprenant un métal choisi parmi le nickel, le rhodium, le palladium et/ou le platine est utilisé.

3. Procédé selon la revendication 2, **caractérisé en ce qu'**un complexe métal-carbène comprenant du palladium en tant que métal est utilisé.

4. Procédé selon au moins l'une quelconque des revendications 1 à 3, **caractérisé en ce qu'**un complexe métal-carbène comprenant au moins un ligand carbène choisi parmi les structures 1 à 4 avec $R_3$ à $R_6$ = hydrogène est utilisé.

5. Procédé selon la revendication 4, **caractérisé en ce qu'**un complexe métal-carbène comprenant au moins un ligand carbène de structure 2 avec $R_3$ et $R_4$ = hydrogène est utilisé.

6. Procédé selon la revendication 5, **caractérisé en ce qu'**un complexe métal-carbène comprenant au moins un ligand carbène de structure 2 avec $R_3$ et $R_4$ = hydrogène et $R_1$ et $R_2$ avec n = 0 et B = un groupe phényle ou 2,4,6-triméthylphényle est utilisé.

7. Procédé selon au moins l'une quelconque des revendications 1 à 6, **caractérisé en ce que** la concentration du catalyseur, formellement donnée en ppm (masse) de métal par rapport à la masse totale du mélange réactionnel, au début de l'hydrodimérisation est de 1 à 500 ppm.

8. Procédé selon au moins l'une quelconque des revendications 1 à 7, **caractérisé en ce que** des composés choisis parmi le 1,3-butadiène, l'isoprène, le 1,3-pentadiène, le chloroprène ou les mélanges correspondants comprenant

des oléfines non cycliques contenant au moins deux doubles liaisons conjuguées sont utilisés en tant qu'oléfines non cycliques contenant au moins deux doubles liaisons conjuguées.

**9.** Procédé selon au moins l'une quelconque des revendications 1 à 8, **caractérisé en ce que** des oléfines non cycliques ne comprenant pas de stabilisateur ou comprenant un stabilisateur choisi parmi les phénols alkylés ou les radicaux N-oxyle stables sont utilisées.

**10.** Procédé selon la revendication 9, **caractérisé en ce que** des stabilisateurs agissant par voie radicalaire, choisis parmi les phénols alkylés ou les radicaux N-oxyle stables, sont utilisés en tant que stabilisateur.

**11.** Procédé selon la revendication 10, **caractérisé en ce que** des radicaux N-oxyle choisis parmi les 2,2,6,6-tétraméthylpipéridine-N-oxyles substitués en position 4 ou non substitués, le 4-hydroxy-2,2,6,6-tétraméthylpipéridine-N-oxyle ou le 2,2,6,6-tétraméthylpipéridine-N-oxyle sont utilisés en tant que radicaux N-oxyle.

**12.** Procédé selon la revendication 8, **caractérisé en ce que** le 1,3-butadiène est utilisé en tant qu'oléfine non cyclique.

**13.** Procédé selon au moins l'une quelconque des revendications 1 à 12, **caractérisé en ce que** l'acide formique est utilisé en tant que réducteur.

**14.** Procédé selon la revendication 13, **caractérisé en ce que** l'acide formique est introduit au cours de la réaction.

**15.** Procédé selon au moins l'une quelconque des revendications 1 à 14, **caractérisé en ce que** l'hydrodimérisation est réalisée en présence de bases.

**16.** Procédé selon la revendication 15, **caractérisé en ce que** des hydroxydes de métaux alcalins, des alcoolates de métaux alcalins ou des amines tertiaires sont utilisés en tant que bases.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0004408 B1
- EP 0004410 B1
- EP 0004410 A
- EP 0007666 B1
- EP 0012472 A
- EP 0012475 A
- EP 0008139 B1
- DE 3024877 A1
- DE 3024878 A1
- DE 3024879 A1
- DE 3034098 A1
- JP 9087207 A
- EP 0704417 A2
- DE 10149347 A1
- WO 2005047217 A
- WO 2005047218 A
- DE 10128144 A1
- DE 4447066
- WO 9734875 A


### In der Beschreibung aufgeführte Nicht-Patentliteratur


- **GARDNER et al.** *Tetrahedron Lett.,* 1972, vol. 2, 163-164
- **ROFFIA et al.** *J. Organomet. Chem.,* 1973, vol. 55, 405-407
- *J. Mol. Catal.,* 1982, vol. 15, 377-381
- *Chem. Eur. J.,* 2004, vol. 10, 3891-3900
- **W. A. HERRMANN ; C. KÖCHER.** *Angew. Chem.,* 1997, vol. 109, 2257
- *Angew. Chem. Int. Ed. Engl.,* 1997, vol. 36, 2162
- **V.P.W. BÖHM ; C.W.K. GSTÖTTMAYR ; T. WESKAMP ; W.A. HERRMANN.** *J. Organomet. Schem.,* 2000, vol. 595, 186
- *Organometallics,* 1999, vol. 18, 529-533